# EUROPÄISCHE PATENTSCHRIFT

(11) **EP 3 687 630 B1**
(45) Veröffentlichungstag und Bekanntmachung des Hinweises auf die Patenterteilung: **06.10.2021**
(21) Anmeldenummer: 18765077.5
(22) Anmeldetag: 31.08.2018
(51) Int. Cl.: A61K 8/893, A61Q 1/02, A61K 8/58, A61K 8/06, A61K 8/02, A61K 8/891

(54) **VERWENDUNG EINER KOSMETISCHEN ZUBEREITUNG ENTHALTEND CETYL DIGLYCERYL TRIS(TRIMETHYLSILOXY)SILYLETHYL DIMETHICONE**
USE OF A COSMETIC PREPARATION CONTAINING CETYL-DIGLYCERYL TRIS(TRIMETHYLSILOXY)SILYLETHYL DIMETHICONE
UTILISATION D'UNE PRÉPARATION COSMÉTIQUE CONTENANT DES CÉTYL DIGLYCÉRYL TRIS(TRIMÉTHYLSILOXY)SILYLÉTHYL DIMÉTHICONES

(30) Priorität: 28.09.2017 DE 102017217287
(43) Veröffentlichungstag der Anmeldung: 05.08.2020
(73) Patentinhaber: Beiersdorf AG, 20253 Hamburg (DE)
(72) Erfinder: BLUCK, Manuela, 21465 Wentorf (DE); KIENAST, Romy, 20251 Hamburg (DE); RIEDEL, Cornelia, 22889 Tangstedt (DE); HEIKE, Kerstin, 22457 Hamburg (DE)
(74) Vertreter: Beiersdorf AG
(86) Internationale Anmeldenummer: PCT/EP2018/073417
(87) Internationale Veröffentlichungsnummer: WO 2019/063239

(56) Entgegenhaltungen:
- EP-A2- 2 033 686
- WO-A1-2017/133822
- DE-A1- 10 238 432
- Cindy Delvall? ET AL: "New Formulation Possibilities with a Water-in-oil Silicone Emulsifier Suitable for PEG-free Systems", , 31. Dezember 2014 (2014-12-31), XP055349098, Gefunden im Internet: URL:http://www.dowcorning.com/content/publ ishedlit/27-1463-water-in-oil-silicone-emu lsifier.pdf [gefunden am 2017-02-23]

## Beschreibung

Die vorliegende Erfindung betrifft eine kosmetische Zubereitung zur Applikation auf die menschliche Haut, insbesondere eine Pflegezubereitung mit farbgebenden Pigmenten, die über ein Pad oder ein Tuch auf die Haut appliziert werden.

Der Wunsch, schön und attraktiv auszusehen, ist schon immer in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Gesichts-Make-up soll der Gesichtshaut ein natürliches Aussehen verleihen, blasse Haut auffrischen und farbliche Unregelmäßigkeiten der Haut ausgleichen.

Neben Gesichtspudern und Rouge als pulverförmigen Kosmetika, werden hierzu cremeförmige Präparate, wie Tagescremes und Creme-Make-up auf Emulsionsbasis verwendet. Eine besondere Ausführungsform ist die so genannte Foundation. Als Foundation bezeichnet man dabei flüssige oder halbfeste Make-up-Präparate, die meist Hautfarben sind und auf das Gesicht, insbesondere auf die Wangen aufgetragen werden. Sie verleihen diesen ein gleichmäßiges gesundes gebräuntes oder rötliches Aussehen.

Foundations können in unterschiedlicher Form aufbewahrt, dargereicht und aufgetragen werden. Neben einfachen Flaschen und Tuben, Überdruck- oder Pumpspendern, kommen in jüngerer Zeit auch Flaschen und Tuben mit aufgesetztem Applikator, insbesondere Pinseln und Schwämmen zum Einsatz. Derartige Systeme haben den großen Vorteil, dass sich die Zubereitungen direkt aus dem Vorratsbehältnis auf die Haut auftragen lassen.

Eine für den Verbraucher besonders attraktive Darreichungsform umfasst ein verschließbares Behältnis mit einer Vertiefung in der sich ein Schwamm befindet. Der Schwamm ist getränkt mit einer kosmetischen Zubereitung und dient somit als Reservoir. Zur Anwendung der kosmetischen Zubereitung auf der Haut, wird ein Wattepad oder ein Papiertuch auf den eingeweichten Schwamm getupft. Dabei sollte das angewendete Wattepad oder Papiertuch die kosmetische Zubereitung aufnehmen. Abschließend wird die kosmetische Zubereitung über das Wattepad oder das Papiertuch auf die Haut appliziert.

Ein derartiges Packmittel und eine derartige Anwendung haben viele Vorteile. Zum einen können diese optisch attraktiv für den Verbraucher gestaltet werden und zum anderen erlaubt die Anwendung des Schwamms als Reservoir in Kombination mit einem Wattepad oder Papiertuch, dass der Verbraucher die kosmetische Zubereitung über das Wattepad oder Papiertuch ohne direkten Kontakt mit den Fingern applizieren kann. Ferner wird durch die Lagerung der kosmetischen Zubereitungen in einem Schwamm verhindert, dass die kosmetische Zubereitung ausläuft, sofern der Schwamm nicht im Packmittel ausgequetscht wird.

Nachteilig am Stand der Technik ist, dass bei Anwendung von Papiertüchern oder Wattepads zur Auftragung einer derartigen kosmetischen Zubereitung die Papiertücher oder Wattepads oftmals unzureichend eingeweicht werden. Demensprechend ist ein Transfer der kosmetischen Zubereitung von dem Schwamm auf ein Papiertuch oder Wattepad recht ineffizient, so dass der Verbraucher oftmals mehrmals auf den Schwamm tupfen muss oder mehr Druck aufwenden muss, um eine ausreichende Menge an kosmetischer Zubereitung aufnehmen zu können. Durch Anwendung einer erhöhten Menge Drucks wird der Schwamm jedoch gequetscht, was zu einem unerwünschten Auslaufen der Zubereitung im Packmittel führt.

Der Begriff "Einweichen" bezieht sich auf die Fähigkeit der kosmetischen Zubereitung, durch die Blätter / Schichten des Cellulose haltigen Applikationsmittels einzudringen, zu durchdringen, zu diffundieren und zu passieren. Je schneller eine kosmetische Zubereitung eindringt, durchdringt, diffundiert und durch die Blätter/Schichten des Applikationsmittels passiert, desto schneller kann die kosmetische Zubereitung vom Applikationsmittel aufgenommen werden. Dementsprechend ist eine leichtere Aufnahme und Applikation auf die Haut möglich, ohne dass eine erhöhte Menge Druck auf den Schwamm ausgeübt werden muss.

Es war daher Aufgabe der vorliegenden Erfindung die Nachteile des Standes der Technik zu beseitigen. Insbesondere war es Aufgabe der Erfindung eine kosmetische Zubereitung, beispielsweise zum Auftragen von Make-up, insbesondere von Foundations, bereitzustellen, welche als Tränkungsmedium für einen Schwamm derart geeignet ist, dass sich die kosmetische Zubereitung bei Aufnahme mit einem Cellulose enthaltenden Tuch oder Pad besonders leicht einweichen lässt.

Die Aufgabe wird überraschend gelöst durch die Verwendung einer kosmetische Zubereitung enthaltend, bezogen auf das Gesamtgewicht der kosmetischen Zubereitung,
a) Cetyl Diglyceryl Tris(Trimethylsiloxy)silylethyl Dimethicone und
b) 8 Gew.-% bis 40 Gew.-% Silikonöle
zur schnelleren Einweichung der Zubereitung in Cellulose enthaltende Applikationsmittel, welche vorteilhaft Tücher und/oder Pads sind.

Auch Gegenstand der Erfindung ist ein Verfahren zur Auftragung einer kosmetischen Zubereitung auf die Haut dadurch gekennzeichnet, dass die kosmetische Zubereitung enthaltend Cetyl Diglyceryl Tris(Trimethylsiloxy)silylethyl Dimethicone und Silikonöle in einem ersten Schritt mit einem Cellulose enthaltenden Applikationsmittel, vorteilhaft mit einem Tuch oder Pad, aufgenommen und die kosmetische Zubereitung in einem zweiten Schritt mit dem Cellulose enthaltenden Applikationsmittel, vorteilhaft mit einem Tuch oder Pad, auf die Haut aufgetragen wird.

Zwar kennt der Fachmann die EP 2033686 A2 und die DE102006028549 A1, doch konnten diese Schriften nicht den Weg zur vorliegenden Erfindung weisen.

Ferner kennt der Fachmann das Dokument WO 2017/133822 A1. welches Zubereitungen mit Cetyl Diglyceryl Tris(Trimethylsiloxy)silylethyl Dimethicone und Silikonölen aufweist.

Das Dokument "New Formulation Possibilities with a Water-in-Oil Silicone Emulsifier Suitable for PEG-free Systems" von Cindy Delvalle & Al offenbart verschiedene Zubereitungen enthaltend Cetyl Diglyceryl Tris(Trimethylsiloxy)silylethyl Dimethicone und Silikonölen.

DE 10238432 A1 offenbart, dass Applikationsmittel aus Cellulose für ihre Saugfähigkeit bekannt sind.

Jedoch konnten auch diese Dokumente keinen Hinweis auf die vorliegende Erfindung liefern.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung und das erfindungsgemäße Verfahren.

Es ist erfindungsgemäß bevorzugt, wenn es sich bei der erfindungsgemäßen kosmetischen Zubereitung um eine Wasser-in-Silikonöl-Emulsion (W/S-Emulsion) handelt.

Alle nachfolgend aufgeführten Gewichtsprozentangaben (Gew.-%) beziehen sich, sofern nicht anders angegeben, jeweils auf das Gesamtgewicht der kosmetischen Zubereitung.

Wenn in der folgenden Beschreibung Verhältnisse bestimmter Komponenten/Substanzen/Stoffgruppen offenbart werden, beziehen sich diese Verhältnisse, sofern nicht anders angegeben, auf Gewichtsverhältnisse der Komponenten/Substanzen/Stoffgruppen.

Der Ausdruck "frei von" bedeutet im Sinne der vorliegenden Offenbarung, dass der Anteil der jeweiligen Substanz kleiner 0,05 Gew.-% ist. Dadurch wird gewährleistet, dass Einschleppungen oder Verunreinigungen mit diesen Stoffen nicht als erfindungsgemäß "frei von" mitumfasst werden.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird in dieser Offenbarung der Ausdruck Tuch oder Pad verwendet, so ist dieses ein Applikationsmittel, welches Cellulosefasern enthält. Tücher und/oder Pads sind dem Fachmann in der dekorativen Kosmetik als übliche kosmetische Applikationsmittel weitreichend bekannt.

Wird in der vorliegenden Offenbarung der Ausdruck "Haut" verwendet so bezieht sich dieser ausschließlich auf die menschliche Haut.

Erfindungsgemäß werden unter Wasser-in-Silikonöl-Emulsion Emulsionen mit einer inneren wässrigen Phase verstanden, deren Ölphase zu mindestens 40 Gew.-% aus Silikonölen besteht, wobei sich die Angabe auf das Gesamtgewicht der Ölphase bezieht. Emulgatoren zählen erfindungsgemäß nicht zur Ölphase.

Der Emulgator Cetyl Diglyceryl Tris(Trimethylsiloxy)silylethyl Dimethicone kann vorteilhaft im Sinne der vorliegenden Erfindung unter der Handelsbezeichnung Dow Corning ® ES-5600 Silicone Glycerol Emulsifier bei der Fa. Dow Corning bezogen werden.

Es ist erfindungsgemäß vorteilhaft, wenn die erfindungsgemäße kosmetische Zubereitung Cetyl Diglyceryl Tris(Trimethylsiloxy)silylethyl Dimethicone in einem Anteil von 0,5 Gew.-% bis 20 Gew.-%, bezogen auf das Gesamtgewicht der kosmetischen Zubereitung, enthält.

Es ist erfindungsgemäß bevorzugt, wenn die erfindungsgemäße kosmetische Zubereitung Cetyl Diglyceryl Tris(Trimethylsiloxy)silylethyl Dimethicone in einem Anteil von 1,5 Gew.-% bis 10 Gew.-%, bezogen auf das Gesamtgewicht der kosmetischen Zubereitung, enthält.

Es ist erfindungsgemäß insbesondere bevorzugt, wenn die erfindungsgemäße kosmetische Zubereitung Cetyl Diglyceryl Tris(Trimethylsiloxy)silylethyl Dimethicone in einem Anteil von 2 Gew.-% bis 6 Gew.-%, bezogen auf das Gesamtgewicht der kosmetischen Zubereitung, enthält.

Ferner ist es erfindungsgemäß vorteilhaft, wenn der Anteil der Silikonöle 10 Gew.-% bis 30 Gew.-%, insbesondere vorteilhaft 12 bis 20 Gew.-%, bezogen auf das Gesamtgewicht der kosmetischen Zubereitung beträgt.

Erfindungsgemäß werden die Silikonöle bevorzugt gewählt aus der Gruppe der Dimethicone und Cyclomethicone. Insbesondere bevorzugt ist es, wenn Dimethicon als Siliconöl enthalten ist.

Es ist dabei erfindungsgemäß weiterhin vorteilhaft, wenn das enthaltene Silikonöl, insbesondere das erfindungsgemäß besonders bevorzugte Dimethicon, eine kinematische Viskosität im Bereich von 0,5 cSt bis 10 cSt bei 25°C aufweist. Die Messung der Viskosität des Silikonöls erfolgt gemäß der Veröffentlichung der American Society for Testing And Materials (ASTM), Philadelphia, Penn., ASTM D 445-94, "Standard Test Method for Kinematic Viscosity of Transparent and Opaque Liquids (the Calculation of Dynamic Viscosity)".

Es ist erfindungsgemäß vorteilhaft, wenn die erfindungsgemäße kosmetische Zubereitung Farbstoffe und/oder Farbpigmente in einer Gesamtmenge von 1 Gew.-% bis 30 Gew.-%, bezogen auf das Gesamtgewicht der kosmetischen Zubereitung, enthält.

Es ist erfindungsgemäß besonders vorteilhaft, wenn die erfindungsgemäße Zubereitung Farbstoffe und/oder Farbpigmente in einer Gesamtmenge von 4 Gew.-% bis 20 Gew.-%, bezogen auf das Gesamtgewicht der Zubereitung, enthält.

Die erfindungsgemäßen Pigmente können anorganisch oder organisch sein.

Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid-Pigmente, Chromoxide), wobei die Pigmente beschichtet ("gecoatet") oder unbeschichtet vorliegen können. Unter den Farbpigmenten sind Eisenoxide besonders bevorzugt.

Weißpigmente sind Pigmente, deren optische Wirkung vorwiegend auf nicht-selektiver Lichtstreuung beruht (siehe auch DIN 55944: 2003-11). Von den chemisch oft sehr ähnlichen Füllstoffen unterscheiden sich anorganische Weißpigmente vor allem durch ihre im Allgemeinen höhere Brechzahl und - damit verbunden - ihr höheres Streuvermögen sowie nach DIN 55943: 2001-10 durch ihre Anwendung.

Erfindungsgemäß bevorzugte Weißpigmente zeigen keine Absorption im Bereich des sichtbaren Lichts, dafür aber ein hohes Streuvermögen, welches ein hohes Deckvermögen zur Folge hat. Das Streuvermögen ist umso größer, je größer die Differenz zwischen der Brechzahl des Weißpigmentes und der des umgebenden Mediums ist.

Erfindungsgemäß vorteilhafte Weißpigmente sind Titandioxide (Brechzahlen: 2,55 für Anatas und 2,75 für Rutil) und Zinkoxide (Brechzahl zwischen 1,95 und 2,1). Besonders bevorzugt ist Titandioxid.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelsübliche Effektpigmente sind: Timiron® von Merck, Iriodin® von Merck (Perl- und Farbglanzpigmente für dekorative technische Anwendungen), Xirallic® von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Es ist ferner vorteilhaft im Sinne der vorliegenden Erfindung, wenn die erfindungsgemäße Zubereitung einen oder mehrere Farbstoffe enthält.

Die Farbstoffe können sowohl synthetischen als auch natürlichen Ursprungs sein.

Es ist erfindungsgemäß vorteilhaft, wenn die erfindungsgemäße kosmetische Zubereitung Glycerin in einer Konzentration von 1 Gew.-% bis 20 Gew.-%, bezogen auf das Gesamtgewicht der kosmetischen Zubereitung, enthält.

Es ist erfindungsgemäß besonders bevorzugt, wenn die erfindungsgemäße kosmetische Zubereitung Glycerin in einer Konzentration von 7,5 Gew.-% bis 12 Gew.-%, bezogen auf das Gesamtgewicht der kosmetischen Zubereitung, enthält.

Erfindungsgemäß vorteilhafte Ausführungsformen der vorliegenden Erfindung sind dadurch gekennzeichnet, dass die erfindungsgemäße Zubereitung ein oder mehrere Füllstoffe enthält.

Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) sowie Lauroyllysine, Polymethylsilesquioxane, Polymethylmethacrylate, Polymethylmethacrylate Crosspolymer, Talkum, Mica und Silica.

Es ist erfindungsgemäß bevorzugt, wenn die erfindungsgemäße kosmetische Zubereitung einen oder mehrere Füllstoffe gewählt aus der Gruppe der Verbindungen der Stärkederivate, insbesondere Tapiocastärke, enthält.

Vorteilhaft im Sinne der vorliegenden Erfindung ist es, wenn die kosmetische Zubereitung Füllstoffe in einer Gesamtmenge von 1 Gew.-% bis 10 Gew.-%, bezogen auf das Gesamtgewicht er kosmetischen Zubereitung, enthält.

Es ist insbesondere vorteilhaft, wenn als Füllstoff Tapiocastärke enthalten ist und der Gesamtanteil der Füllstoffe 1,5 Gew.-% bis 4 Gew.-% beträgt.

Ferner ist es vorteilhaft im Sinne der vorliegenden Erfindung, wenn die erfindungsgemäße kosmetische Zubereitung mindestens ein hydrophobiertes pyrogeniertes Siliciumdioxid enthält.

Mit "pyrogeniertem Siliciumdioxid", das auch mit "pyrogenem Siliciumdioxid" oder "sublimiertem Siliciumdioxid" bezeichnet wird, ist ein Siliciumdioxid gemeint, das durch Hydrolyse von Chlorsilanen hergestellt wird. Dieses wird hydrophob mit Dimethyldichlorsilan oder mit Hexamethyldisiloxan behandelt, wobei Dimethyldichlorsilan bevorzugt ist. Das erhaltene hydrophobierte Siliciumdioxid weist vorzugsweise eine mittlere Teilchengröße von 5 bis 20 nm, be-- vorzugter von 10 bis 20 nm und / oder eine spezifische Oberfläche von 50 bis 300 m²/g, bevorzugter von 80 bis 140 m²/g auf.

Beispiele für hydrophobierte pyrogenierte Kieselsäuren sind diejenigen, die unter den INCI-Namen von Silica Silylate und Silica Dimethyl Silylate bezeichnet werden. Kieselsäuren dieser Art werden vor allem von Evonik unter den Handelsnamen AEROSIL® R812 und AEROSIL® R 972 vermarktet.

Der Gesamtanteil des hydrophobierten pyrogenierten Siliciumdioxids beträgt vorteilhaft 0,5 Gew.-% bis 2 Gew.-%, bezogen auf das Gesamtgewicht der kosmetischen Zubereitung.

Überraschend hat sich ferner herausgestellt, dass bei Anwendung des nachfolgend beschriebenen Herstellungsverfahrensmerkmals eine kosmetische Zubereitung erhalten werden konnte, die nicht ausklumpt und bei der eine reduzierte Menge an weißen Rückständen in der kosmetischen Zubereitung und nach Auftragung auf der Haut aufgefunden wurden. Eine derartige Zubereitung wurde erhalten, indem in einem ersten Schritt die Tapiokastärke in Glycerin dispergiert wurde und die Dispersion in einem zweiten Schritt der wässrigen Phase und der Ölphase der kosmetischen Zubereitung zugegeben wurde. Wird hingegen die Tapiokastärke direkt der Emulsion zugegeben ohne eine vorherige Dispersion in Glycerin, so fällt diese beim Auftragen oder bereits bei Herstellung der kosmetischen Zubereitung aus.

Die erfindungsgemäße Zubereitung kann darüber hinaus ein oder mehrere UV-Lichtschutzfilter und/oder kosmetische Wirkstoffe enthalten.

Erfindungsgemäß vorteilhafte Ausführungsformen der vorliegenden Erfindung sind dadurch gekennzeichnet, dass die kosmetische Zubereitung einen oder mehrere UV-Filter gewählt aus der Gruppe der Verbindungen Phenylen-1,4-bis-(2-benzimidazyl)-3,3'-5,5'-tetrasulfon-säuresalze; 2-Phenylbenzimidazol-5-sulfonsäuresalze; 1,4-di(2-oxo-10-Sulfo-3-bornyliden-methyl)-Benzol und dessen Salze; 4-(2-Oxo-3-bornylidenmethyl)benzolsulfonsäuresalze; 2-Methyl-5-(2-oxo-3-borny-lidenmethyl)sulfonsäuresalze; 2,2'-Methylen-bis-(6-(2H-benzotriazol-2-yl)-4-(1,1,3,3-tetramethylbutyl)-phenol) (Handelsname: Tinosorb M); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-cyano-3,3-diphenylacrylat; Dimethico-diethylbenzalmalonat; 3-(4-(2,2-bis Ethoxycarbonylvinyl)-phenoxy)propenyl)-methoxysiloxan / Dimethylsiloxan - Copolymer (INCI: Polysilicone-15); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); Dioctylbutylamidotriazon (INCI: Diethylhexyl-Butamidotriazone); 2,4-bis-[5-1(dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäuretris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; den Merocyaninverbindungen; in einem Anteil von 0,01 Gew.-% bis 40 Gew.-% bezogen auf das Gesamtgewicht der kosmetischen Zubereitung enthält. Dabei sind Konzentrationen von 1 Gew.-% bis 20 Gew.-% und insbesondere 5 Gew.-% bis 10 Gew.-%, bezogen auf das Gesamtgewicht der Zubereitung erfindungsgemäß bevorzugt.

Erfindungsgemäß bevorzugt enthält die erfindungsgemäße kosmetische Zubereitung organische Carbonate, insbesondere Propylencarbonat und/oder Butylencarbonat, wobei der Einsatz von Propylencarbonat erfindungsgemäß besonders bevorzugt ist.

Derartige organische Carbonate, insbesondere Propylencarbonat, werden erfindungsgemäß vorteilhaft in einer Konzentration von 0,1 Gew.-% bis 0,7 Gew.-%, bezogen auf das Gesamtgewicht der kosmetischen Zubereitung, eingesetzt.

Darüber hinaus ist es erfindungsgemäß weiterhin vorteilhaft, wenn die kosmetische Zubereitung zur weiteren Stabilisierung mindestens ein Aminosäuretensid enthält. Erfindungsgemäß bevorzugte Aminosäuretenside sind gewählt aus der Gruppe der Glutamate, wie beispielsweise das unter der INCI Bezeichnung Sodium Stearoyl Glutamate. Aminosäuretenside, insbesondere bevorzugt Sodium Stearoyl Glutamate, sind vorteilhaft in einem Gesamtanteil von 0,01 Gew.-% bis 0,2 Gew.-%, bezogen auf das Gesamtgewicht der kosmetischen Zubereitung, in der erfindungsgemäßen kosmetischen Zubereitung enthalten.

Vorteilhafte Ausführungsformen der vorliegenden Erfindung sind dadurch gekennzeichnet, dass die kosmetische Zubereitung Ethylhexylglycerin und/oder Phenoxyethanol in einer Gesamtkonzentration von 0,8 bis 2 Gew.-%, bezogen auf das Gesamtgewicht der kosmetischen Zubereitung, enthält. Dabei ist es insbesondere vorteilhaft, wenn die Gesamtkonzentration von Ethylhexylglycerin und Phenoxyethanol mindestens 1,1 Gew.-% beträgt und das Gewichtsverhältnis von Ethylhexylglycerin zu Phenoxyethanol 1:1 bis 1:2, insbesondere 1:1,2 bis 1:1,8 beträgt.

Ferner enthalten erfindungsgemäß bevorzugte kosmetische Zubereitungen Wasser in einem Anteil von 30 Gew.-% bis 55 Gew.-%, insbesondere bevorzugt 35 Gew.-% bis 45 Gew.-%, bezogen auf das Gesamtgewicht der kosmetischen Zubereitung.

Erfindungsgemäß vorteilhaft enthält die erfindungsgemäße kosmetische Zubereitung eine oder mehrere Verbindungen gewählt aus der Gruppe der Verbindungen Folsäure, D-Biotin, Coenzym Q10, alpha-Glucosylrutin, Carnitin, natürliche und/oder synthetische Isoflavonoide, Genistein, Flavonoide, Carotinoide, Kreatin, Kreatinin, Taurin, Ascorbinsäure und Derivate, Tocopherol und seine Ester.

Es ist weiterhin vorteilhaft, wenn die kosmetische Zubereitung weitere Rheologiemodifizierer, insbesondere Natriumchlorid, enthält. Der Anteil an Natriumchlorid beträgt vorteilhaft 0,5 Gew.-% bis 2 Gew.-%, bevorzugt 0,8 Gew.-% bis 1,5 Gew.-%, bezogen auf das Gesamtgewicht der kosmetischen Zubereitung.

Ferner ist es vorteilhaft im Sinne der vorliegenden Erfindung, wenn die kosmetische Zubereitung weitere Öl- und Wachskomponenten enthält.

Die erfindungsgemäße kosmetische Zubereitung wird vorteilhaft in einem Behälter aus Kunststoff gelagert. Erfindungsgemäß bevorzugt weist der Behälter aus Kunststoff innen eine Einbuchtung auf, in der ein Schwamm gelagert wird. Vorteilhaft dient der Schwamm als Reservoir für die kosmetische Zubereitung. Überraschend hat sich gezeigt, dass die kosmetische Zubereitung sich besonders effektiv in dem Schwamm lagern lässt, so dass die kosmetische Zubereitung nicht aus dem Packmittel und dem Schwamm herausläuft. Eine unnötige Verschmutzung des Packmittels wird durch die erfindungsgemäß vorteilhafte Lagerung der kosmetischen Zubereitung im Schwamm überraschend vermieden.

Der eingesetzte Schwamm besteht erfindungsgemäß vorteilhaft aus Polyester. Ferner weist der eingesetzte Schwamm vorteilhaft ein Flächengewicht von 100 g/m² bis 300 g/m², bevorzugt 130 g/m² bis 180 g/m² auf.

Da die Anwendung eines durchtränkten Schwammes für die Auftragung von Make-Up eher ungeeignet ist, da durch das Anfassen des Schwammes die kosmetische Zubereitung mit den Fingern in Kontakt kommt, ist es erfindungsgemäß vorteilhaft, wenn die kosmetischen Zubereitung mit einem Cellulose enthaltenden Applikationsmittel, vorteilhaft einem Tuch und/oder Pad, vom Schwamm durch Kontakt aufgenommen und anschließend über das Applikationsmittel auf die Haut übertragen wird. Durch eine derartige vorteilhafte Anwendung, lässt sich die erfindungsgemäße Zubereitung besonders gleichmäßig auftragen. Verschmutzungen an den Fingern werden vermieden.

Der Behälter aus Kunststoff lässt sich vorteilhaft über einen Verriegelungsmechanismus schließen, so dass der Schwamm bei Transport nicht aus dem Behälter fallen kann.

Die nachfolgenden Vergleichsversuche sollen die vorteilhafte Wirkung der vorliegenden Erfindung verdeutlichen.

Dazu wurden die folgenden kosmetischen Zubereitungen bereitgestellt. Alle Mengenangaben, Anteile und Prozentanteile sind, soweit nicht anders angegeben, auf das Gewicht und die Gesamtmenge bzw. auf das Gesamtgewicht der Zubereitungen bezogen:

| **INCI Bezeichnung** | **Bsp. 1** | **Bsp. 2** |
|---|---|---|
| Trisodium EDTA | 0,2 | 0,2 |
| Ethylhexyl Stearate | 5,0 | 5,0 |
| Phenoxyethanol | 0,6 | 0,6 |
| Butyl Methoxydibenzoylmethane | 2,0 | 2,0 |
| Phenylbenzimidazole Sulfonic Acid | 1,0 | 1,0 |
| Glycerin | 8,9 | 8,9 |
| Sodium Hydroxide | 0,15 | 0,15 |
| Cetyl PEG/PPG-10/1 Dimethicone | - | 4,0 |
| Ethylhexyl Salicylate | 4,0 | 4,0 |
| Sodium Chloride | 1,0 | 1,0 |
| Disteardimonium Hectorite | 1,2 | 1,2 |
| Cl 77492 | 0,83 | 0,83 |
| Cl 77491 | 0,21 | 0,21 |
| Cl 77499 | 0,09 | 0,09 |
| Silica Dimethyl Silylate | 1,0 | 1,0 |
| Ethylhexylglycerin | 0,25 | 0,25 |
| Propylene Carbonate | 0,36 | 0,36 |
| Octocrylene | 2,0 | 2,0 |
| Tapioca Starch | 2,6 | 2,6 |
| Cl 77891 | 5,9 | 5,9 |
| Dicaprylyl Carbonate | 2,0 | 2,0 |
| Sodium Stearoyl Glutamate | 0,1 | 0,1 |
| Diethylhexyl Syringylidenemalonate | 0,09 | 0,09 |
| Caprylic/Capric Triglyceride' | 0,01 | 0,01 |
| Sodium Hyaluronate, Magnolia Officinalis Bark Extract, 4-Butylresorcinol, 1-Methylhydantoin-2-Imide, Creatine | 0,2 | 0,2 |
| Dimethicone¹ | 15,0 | 15,0 |
| Cetyl Diglyceryl Tris(Trimethylsiloxy)silylethyl Dimethicone | 4,0 | - |
| Parfum | 0,4 | 0,4 |
| Aqua | Ad 100 | Ad 100 |

| | | |
|---|---|---|
| ¹ Silikon BRB DM5, BRB Chemicals International 4,5 cSt-5,5 cSt Zum Vergleich der erfindungsgemäßen Zubereitung Bsp. 1 und der nicht erfindungsgemäßen Zubereitung Bsp.2 wurde jeweils die Zeit gemessen, die notwendig war, um ein Papiertuch bei Kontakt mit der Zubereitung einzuweichen. | | |

Es wurde der folgende Versuchsaufbau vorgenommen:
Ein oder mehrere Papiertücher der Handelsbezeichnung Kimtech Science Labortücher - 200 Tücher / Weiß - 7558 wurden auf eine Glasplatte gelegt. Anschließend wurde eine definierte Menge der jeweiligen Zubereitung mit einer Pipette auf das Tuch gegeben und die Aufnahmegeschwindigkeit der Zubereitung ermittelt, indem die Zeit gestoppt wurde bis die Flüssigkeit durch das Papiertuch gedrungen war und auf der Glasscheibe Tropfen/Feuchtigkeit erschien(en). Dazu wurde die Glasplatte von unten beobachtet. In der nachfolgenden Tabelle sind die gemessenen Zeiten und Versuchsbedingungen aufgeführt.

| Anzahl der Papiertücher | Zugegebene Menge der Zubereitungen | Zeit in s von Bsp.1 | Zeit in s von Bsp.2 |
|---|---|---|---|
| 1 | 25 µl | 5 | 12 |
| 2 | 150 µl | 67 | Kein durchdringen nach 120 s |

Wie sich aus den Vergleichsversuchen ergibt, werden die Papiertücher von der Bsp.1 deutlich schneller durchdrungen, als von Bsp. 2. Demensprechend ist eine deutlich schnellere und einfachere Aufnahme der Zubereitung möglich.

### Beispiele

Die nachfolgenden Beispiele sollen die vorliegende Erfindung weiter verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | Bsp. 3 | Bsp. 4 | Bsp. 5 | Bsp. 6 | Bsp. 7 |
|---|---|---|---|---|---|
| Trisodium EDTA | 0,2 | 0,5 | 0,2 | 0,5 | 0,2 |
| Ethylhexyl Stearate | 8,0 | 2,0 | 3,0 | 7,0 | - |
| Phenoxyethanol | 0,8 | 0,6 | 0,6 | 0,5 | 0,9 |
| Diethylamino Hydroxybenzoyl Hexyl Benzoate | 3,0 | - | - | 1,5 | - |
| Caprylic/Capric Triglyceride | | 1,0 | 2,0 | | |
| Dibutyl Adipate | | | | | 4,0 |
| Butyl Methoxydibenzoylmethane | | 1,0 | 2,5 | 3 | 0,5 |
| Phenylbenzimidazole Sulfonic Acid | 1,0 | 0,5 | 1,5 | 2,0 | 1,0 |
| Glycerin | 9,0 | 7,0 | 5,0 | 12,0 | 8,0 |
| Sodium Hydroxide | 0,16 | 0,20 | 0,1 | 0,16 | 0,30 |
| Ethylhexyl Triazone | | | | | |
| Ethylhexyl Salicylate | 4,0 | 2,0 | 1,0 | 4,5 | 5,0 |
| Sodium Chloride | 2,0 | 0,5 | 0,8 | 0,2 | 0,1 |
| Bis-Ethylhexyloxyphenol Methoxyphenyl Triazine | - | - | - | 2,5 | - |
| Disteardimonium Hectorite | 0,8 | 0,5 | 0,2 | 1,5 | 0,46 |
| Cl 77492 | 0,5 | 1,5 | 0,40 | 2,0 | 0,2 |
| Cl 77491 | 0,3 | 0,10 | 0,10 | 0,08 | 0,7 |
| Cl 77499 | 0,08 | 0,005 | 0,02 | 0,15 | 1,0 |
| Silica Dimethyl Silylate | 1,0 | 0,5 | 2,0 | 2,5 | 0,3 |
| Ethylhexylglycerin | 0,2 | 0,3 | 0,15 | | 0,5 |
| Propylene Carbonate | 0,2 | 0,1 | 0,15 | 0,27 | 0,5 |
| Octocrylene | 3,0 | 3,0 | 1,0 | - | 1,2 |
| Tapioca Starch | 5,0 | 1,0 | 0,2 | 3,0 | 0,5 |
| Cl 77891 | 1,9 | 9,0 | 1,0 | 6,0 | 3,0 |
| Tin Oxide | 0,7 | - | 0,4 | - | - |
| Synthetic Fluorphlogopite | 5,0 | - | 2,88 | - | - |
| Dicaprylyl Carbonate | 4,0 | 3,0 | 5,0 | 1,8 | 8,0 |
| Sodium Stearoyl Glutamate | 0,1 | 2,0 | 1,5 | 1,1 | - |
| Dimethicone | 12,0 | 10,0 | 18,0 | 16,0 | 15,0 |
| Cetyl Diglyceryl Tris(Trimethylsiloxy)silylethyl Dimethicone | 2,0 | 4,0 | 6,0 | 3,5 | 1,2 |
| Benzethonium Cloride | - | - | - | 0,03 | - |
| Parfum | 0,4 | 0,3 | 0,2 | 0,5 | 0,4 |
| Aqua | Ad 100 | Ad 100 | Ad 100 | Ad 100 | Ad 100 |

## Patentansprüche

1. Verwendung einer kosmetischen Zubereitung enthaltend, bezogen auf das Gesamtgewicht der kosmetischen Zubereitung,
a. Cetyl Diglyceryl Tris(Trimethylsiloxy)silylethyl Dimethicone und
b. 8 Gew.-% bis 40 Gew.-% Silikonöle
zur schnelleren Einweichung der Zubereitung in Cellulose enthaltende Applikationsmittel, welche vorteilhaft Tücher und/oder Pads sind.

2. Verwendung nach Anspruch 1 **dadurch gekennzeichnet, dass** die kosmetische Zubereitung **dadurch gekennzeichnet ist, dass** es sich bei um eine Wasser-in-Silikonöl-Emulsion handelt.

3. Verwendung nach einem der vorhergehenden Ansprüche **dadurch gekennzeichnet, dass** die kosmetische Zubereitung **dadurch gekennzeichnet ist, dass** Cetyl Diglyceryl Tris(Trimethylsiloxy)silylethyl Dimethicone in einem Anteil von 0,5 Gew.-% bis 20 Gew.-%, bezogen auf das Gesamtgewicht der kosmetischen Zubereitung, enthalten ist.

4. Verwendung nach einem der vorhergehenden Ansprüche **dadurch gekennzeichnet, dass** die kosmetische Zubereitung **dadurch gekennzeichnet ist, dass** Cetyl Diglyceryl Tris(Trimethylsiloxy)silylethyl Dimethicone in einer Konzentration von 2 Gew.-% bis 6 Gew.-%, bezogen auf das Gesamtgewicht der kosmetischen Zubereitung, enthalten ist.

5. Verwendung nach einem der vorhergehenden Ansprüche **dadurch gekennzeichnet, dass** die kosmetische Zubereitung **dadurch gekennzeichnet ist, dass** der Anteil der Silikonöle 10 Gew.-% bis 30 Gew.-%, insbesondere vorteilhaft 12 bis 20 Gew.-%, bezogen auf das Gesamtgewicht der kosmetischen Zubereitung beträgt.

6. Verwendung nach einem der vorhergehenden Ansprüche **dadurch gekennzeichnet, dass** die kosmetische Zubereitung **dadurch gekennzeichnet ist, dass** Farbstoffe und/oder Farbpigmente in einer Gesamtmenge von 1 Gew.-% bis 30 Gew.-%, bevorzugt 4 Gew.-% bis 20 Gew.-%, bezogen auf das Gesamtgewicht der kosmetischen Zubereitung, enthalten sind.

7. Verwendung nach einem der vorhergehenden Ansprüche **dadurch gekennzeichnet, dass** die kosmetische Zubereitung **dadurch gekennzeichnet ist, dass** ein oder mehrere Füllstoffe gewählt aus der Gruppe der Verbindungen der Stärkederivate, insbesondere Tapiocastärke, enthalten sind.

8. Verwendung nach einem der vorhergehenden Ansprüche **dadurch gekennzeichnet, dass** die kosmetische Zubereitung **dadurch gekennzeichnet ist, dass** mindestens ein hydrophobiertes pyrogeniertes Siliciumdioxid enthalten ist.

9. Verwendung nach einem der vorhergehenden Ansprüche **dadurch gekennzeichnet, dass** die kosmetische Zubereitung **dadurch gekennzeichnet ist, dass** ein oder mehrere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-cyano-3,3-diphenylacrylat; Dimethico-diethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin; Dioctylbutylamidotriazon; 2,4-bis-[5-1(dimethyl-propyl)benzoxazol-2-yl-(4-phenyl)-imino]-6-(2-ethylhexyl)-imino-1,3,5-triazin mit der CAS Nr. 288254-16-0; 4,4',4"-(1,3,5-Triazin-2,4,6-triyltriimino)-tris-benzoesäure-tris(2-ethylhexylester); 2,4,6-Tribiphenyl-4-yl-1,3,5-triazin; den Merocyaninverbindungen; in einem Anteil von 0,01 Gew.-% bis 40 Gew.-%, bezogen auf das Gesamtgewicht der kosmetischen Zubereitung, enthalten sind.

10. Verwendung nach einem der vorhergehenden Ansprüche **dadurch gekennzeichnet, dass** die kosmetische Zubereitung **dadurch gekennzeichnet ist, dass** organische Carbonate, insbesondere Propylencarbonat, in einer Konzentration von 0,1 Gew.-% bis 0,7 Gew.-%, bezogen auf das Gesamtgewicht der kosmetischen Zubereitung, enthalten sind.

11. Verwendung nach einem der vorhergehenden Ansprüche **dadurch gekennzeichnet, dass** die kosmetische Zubereitung **dadurch gekennzeichnet ist, dass** Aminosäuretenside, bevorzugt Sodium Stearoyl Glutamate, in einem Gesamtanteil von 0,01 Gew.-% bis 0,2 Gew.-%, bezogen auf das Gesamtgewicht der kosmetischen Zubereitung, in der erfindungsgemäßen kosmetischen Zubereitung enthalten sind.

12. Verwendung nach einem der vorhergehenden Ansprüche **dadurch gekennzeichnet, dass** die kosmetische Zubereitung **dadurch gekennzeichnet ist, dass** die kosmetische Zubereitung Ethylhexylglycerin und/oder Phenoxyethanol in einer Gesamtkonzentration von 0,8 bis 2 Gew.-%, bezogen auf das Gesamtgewicht der kosmetischen Zubereitung, enthält.

13. Verwendung nach einem der vorhergehenden Ansprüche **dadurch gekennzeichnet, dass** die kosmetische Zubereitung **dadurch gekennzeichnet ist, dass** Wasser in einem Anteil von 30 Gew.-% bis 55 Gew.-%, insbesondere bevorzugt 35 Gew.-% bis 45 Gew.-%, bezogen auf das Gesamtgewicht der kosmetischen Zubereitung, enthalten ist.

14. Verfahren zur Auftragung einer kosmetischen Zubereitung auf die Haut **dadurch gekennzeichnet, dass** die kosmetische Zubereitung enthaltend Cetyl Diglyceryl Tris(Trimethylsiloxy)silylethyl Dimethicone und Silikonöle in einem ersten Schritt mit einem Cellulose enthaltenden Applikationsmittel, vorteilhaft mit einem Tuch oder Pad, aufgenommen und die kosmetische Zubereitung in einem zweiten Schritt mit dem Cellulose enthaltenden Applikationsmittel, vorteilhaft mit einem Tuch oder Pad, auf die Haut aufgetragen wird.

## Claims

1. Use of a cosmetic preparation comprising, based on the total weight of the cosmetic preparation,
a. cetyl diglyceryl tris(trimethylsiloxy)silylethyl dimethicone and
b. 8% by weight to 40% by weight silicone oils for faster soaking of the preparation into application means containing cellulose,
which are advantageously wipes and/or pads.

2. Use according to Claim 1, **characterized in that** the cosmetic preparation is **characterized in that** said preparation is a water-in-silicone oil emulsion.

3. Use according to either of the preceding claims, **characterized in that** the cosmetic preparation is **characterized in that** cetyl diglyceryl tris(trimethylsiloxy)silylethyl dimethicone is present at a proportion of 0.5% by weight to 20% by weight, based on the total weight of the cosmetic preparation.

4. Use according to either of the preceding claims, **characterized in that** the cosmetic preparation is **characterized in that** cetyl diglyceryl tris(trimethylsiloxy)silylethyl dimethicone is present at a concentration of 2% by weight to 6% by weight, based on the total weight of the cosmetic preparation.

5. Use according to any of the preceding claims, **characterized in that** the cosmetic preparation is **characterized in that** the proportion of silicone oils is 10% by weight to 30% by weight, particularly advantageously 12% to 20% by weight, based on the total weight of the cosmetic preparation.

6. Use according to any of the preceding claims, **characterized in that** the cosmetic preparation is **characterized in that** dyes and/or colour pigments are present at a total amount of 1% by weight to 30% by weight, preferably 4% by weight to 20% by weight, based on the total weight of the cosmetic preparation.

7. Use according to any of the preceding claims, **characterized in that** the cosmetic preparation is **characterized in that** one or more fillers are present selected from the group of compounds of starch derivatives, especially tapioca starch.

8. Use according to any of the preceding claims, **characterized in that** the cosmetic preparation is **characterized in that** at least one hydrophobized fumed silicon dioxide is present.

9. Use according to any of the preceding claims, **characterized in that** the cosmetic preparation is **characterized in that** one or more UV filters are present at a proportion of 0.01% by weight to 40% by weight, based on the total weight of the cosmetic preparation, selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; 4-(tert-butyl)-4'-methoxydibenzoylmethane; homomenthyl salicylate; 2-ethylhexyl 2-cyano-3,3-diphenylacrylate; dimethicodiethylbenzalmalonate; 3-(4-(2,2-bis ethoxycarbonylvinyl)phenoxy)propenyl)methoxysiloxa ne / dimethylsiloxane - copolymer; 2,4-bis-{[4-(2-ethylhexyloxy)-2-hydroxy]phenyl}-6-(4-methoxyphenyl)-1,3,5-triazine; dioctylbutylamidotriazone; 2,4-bis[5-1(dimethylpropyl)benzoxazol-2-yl-(4-phenyl)imino]-6-(2-ethylhexyl)imino-1,3,5-triazine with CAS No. 288254-16-0; tris(2-ethylhexyl) 4,4',4"-(1,3,5-triazine-2,4,6-triyltriimino)trisbenzoate; 2,4,6-tribiphenyl-4-yl-1,3,5-triazine; the merocyanine compounds.

10. Use according to any of the preceding claims, **characterized in that** the cosmetic preparation is **characterized in that** organic carbonates, particularly propylene carbonate, are present at a concentration of 0.1% by weight to 0.7% by weight, based on the total weight of the cosmetic preparation.

11. Use according to any of the preceding claims, **characterized in that** the cosmetic preparation is **characterized in that** amino acid surfactants, preferably sodium stearoyl glutamate, are present in the cosmetic preparation according to the invention at a total fraction of 0.01% by weight to 0.2% by weight, based on the total weight of the cosmetic preparation.

12. Use according to any of the preceding claims, **characterized in that** the cosmetic preparation is **characterized in that** the cosmetic preparation comprises ethylhexylglycerin and/or phenoxyethanol at a total concentration of 0.8% to 2% by weight, based on the total weight of the cosmetic preparation.

13. Use according to any of the preceding claims, **characterized in that** the cosmetic preparation is **characterized in that** water is present at a proportion of 30% by weight to 55% by weight, particularly preferably 35% by weight to 45% by weight, based on the total weight of the cosmetic preparation.

14. Method for applying a cosmetic preparation to the skin, **characterized in that** the cosmetic preparation comprising cetyl diglyceryl tris(trimethylsiloxy)silylethyl dimethicone and silicone oils is absorbed in a first step with an application means containing cellulose, advantageously with a wipe or pad, and the cosmetic preparation is applied to the skin in a second step using the application means containing cellulose, advantageously with a wipe or pad.

## Revendications

1. Utilisation d'une préparation cosmétique contenant, par rapport au poids total de la préparation cosmétique,
a. des cétyl diglycéryl tris(triméthylsiloxy)silyl-éthyl diméthicones et
b. 8 % en poids à 40 % en poids d'huiles de silicone pour imprégner plus rapidement la préparation dans des agents d'application contenant de la cellulose, qui avantageusement sont des linges et/ou des tampons.

2. Utilisation selon la revendication 1, **caractérisée en ce que** la préparation cosmétique est **caractérisée en ce qu'**il s'agit d'une émulsion eau-dans-l'huile de silicone.

3. Utilisation selon l'une des revendications précédentes, **caractérisée en ce que** la préparation cosmétique est **caractérisée en ce qu'**elle contient des cétyl diglycéryl tris(triméthylsiloxy)silyléthyl diméthicones selon une portion de 0,5 % en poids à 20 % en poids par rapport au poids total de la préparation cosmétique.

4. Utilisation selon l'une des revendications précédentes, **caractérisée en ce que** la préparation cosmétique est **caractérisée en ce qu'**elle contient des cétyl diglycéryl tris(triméthylsiloxy)silyléthyl diméthicones à une concentration de 2 % en poids à 6 % en poids par rapport au poids total de la préparation cosmétique.

5. Utilisation selon l'une des revendications précédentes, **caractérisée en ce que** la préparation cosmétique est **caractérisée en ce que** la proportion des huiles de silicone est de 10 % en poids à 30 % en poids, d'une manière particulièrement avantageuse de 12 à 20 % en poids, par rapport au poids total de la préparation cosmétique.

6. Utilisation selon l'une des revendications précédentes, **caractérisée en ce que** la préparation cosmétique est **caractérisée en ce qu'**elle contient des colorants et/ou des pigments colorés, en une quantité totale de 1 % en poids à 30 % en poids, de préférence de 4 % en poids à 20 % en poids, par rapport au poids total de la préparation cosmétique.

7. Utilisation selon l'une des revendications précédentes, **caractérisée en ce que** la préparation cosmétique est **caractérisée en ce qu'**elle contient une ou plusieurs charges choisies dans le groupe des composés et des dérivés de l'amidon, en particulier l'amidon de tapioca.

8. Utilisation selon l'une des revendications précédentes, **caractérisée en ce que** la préparation cosmétique est **caractérisée en ce qu'**elle contient au moins un dioxyde de silicium pyrogéné hydrophobisé.

9. Utilisation selon l'une des revendications précédentes, **caractérisée en ce que** la préparation cosmétique est **caractérisée en ce qu'**elle contient un ou plusieurs filtres UV choisis dans le groupe des composés sels de l'acide phénylène-1,4-bis-(2-benzimidazyl)-3,3',5,5'-tétrasulfonique ; sels de l'acide 2-phényl-benzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disilanoxyl]propyl]-phénol ; 3-(4-méthylbenzylidène)camphre ; 3-benzylidènecamphre; salicylate d'éthylhexyle; acide téréphtalidènedicamphosulfonique ; ester 2-éthylhexylique de l'acide 4-(diméthylamino)-benzoïque; ester amylique de l'acide 4-(diméthylamino)benzoïque; ester di(2-éthylhexylique) de l'acide 4-méthoxybenzalmalonique; ester 2-éthylhexylique de l'acide 4-méthoxycinnamique; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque, 4-(tert-butyl)-4'-méthoxydibenzoylméthane ; salicylate d'homomenthyle ; acrylate de 2-éthylhexyl-2-cyano-3,3-diphényle ; benzalmalonate de diméthico-diéthyle; copolymère 3-(4-(2,2-biséthoxycarbonylvinyl)-phénoxy)propényl)-méthoxysiloxane/diméthylsiloxane ; 2,4-bis-{[4-(2-éthyl-hexyloxy)-2-hydroxy]-phényl}-6-(4-méthoxyphényl)-1,3,5-triazine ; dioctylbutylamidotriazone ; 2,4-bis-[5-1(diméthyl-propyl)benzoxazol-2-yl-(4-phényl)-imino]-6-(2-éthylhexyl)-imino-1,3,5-triazine portant le numéro CAS 288254-16-0 ; ester tris(2-éthylhexylique) de l'acide 4,4',4"-(1,3,5-triazine-2,4,6-triyltriimino)-tris-benzoïque; 2,4,6-tribiphényl-4-yl-1,3,5-triazine ; les composés mérocyanines ; selon une proportion de 0,01 % en poids à 40 % en poids, par rapport au poids total de la préparation cosmétique.

10. Utilisation selon l'une des revendications précédentes, **caractérisée en ce que** la préparation cosmétique est **caractérisée en ce qu'**elle contient des carbonates organiques, en particulier des propylènecarbonates, à une concentration de 0,1 % en poids à 0,7 % en poids, par rapport au poids total de la préparation cosmétique.

11. Utilisation selon l'une des revendications précédentes, **caractérisée en ce que** la préparation cosmétique est **caractérisée en ce que** la préparation cosmétique selon l'invention contient des tensioactifs à base d'acides aminés, de préférence du stéaroylglutamate de sodium, selon une proportion totale de 0,01 % en poids à 0,2 % en poids, par rapport au poids total de la préparation cosmétique.

12. Utilisation selon l'une des revendications précédentes, **caractérisée en ce que** la préparation cosmétique est **caractérisée en ce que** la préparation cosmétique contient de l'éthylhexylglycérol et/ou du phénoxyéthanol à une concentration totale de 0,8 à 2 % en poids, par rapport au poids total de la préparation cosmétique.

13. Utilisation selon l'une des revendications précédentes, **caractérisée en ce que** la préparation cosmétique est **caractérisée en ce qu'**elle contient de l'eau selon une proportion de 30 % en poids à 55 % en poids, d'une manière particulièrement préférée de 35 % en poids à 45 % en poids, par rapport au poids total de la préparation cosmétique.

14. Procédé d'application d'une préparation cosmétique sur la peau, **caractérisé en ce que** la préparation cosmétique contenant des cétyl diglycéryl tris(triméthylsiloxy)silyléthyl diméthicones et des huiles de silicone est dans une première étape prise avec un agent d'application contenant de la cellulose, de préférence un linge ou un tampon, et, dans une deuxième étape, on applique sur la peau la préparation cosmétique avec l'agent d'application contenant de la cellulose, de préférence avec un linge ou un tampon.
